# EUROPEAN PATENT SPECIFICATION

(11) **EP 4 643 822 B1**
(45) Date of publication and mention of the grant of the patent: **02.12.2026**
(21) Application number: 25172787.1
(22) Date of filing: 28.04.2025
(51) Int. Cl.: A61F 2/30, A61B 17/17, A61B 17/80, A61F 2/36

(54) **FRACTURE FIXATION SYSTEM FOR LONG BONE**
FRAKTURFIXATIONSSYSTEM FÜR LANGE KNOCHEN
SYSTÈME DE FIXATION DE FRACTURE POUR OS LONG

(30) Priority: 29.04.2024 US 202463639773 P
(43) Date of publication of application: 05.11.2025
(73) Proprietor: Howmedica Osteonics Corp., Mahwah, NJ 07430 (US)
(72) Inventor: TULKIS, Peter, Paramus, 07652 (US); SUBHASHIS, Shaw, 700028 Calcutta (IN); CHON, Sanggu Simon, Little Ferry, 07643 (US); WESTRICH, Geoffrey, New York, 10028 (US)
(74) Representative: Regimbeau

(56) References cited:
- US-A- 5 658 349
- US-A1- 2011 009 973
- US-A1- 2014 200 619
- US-A1- 2023 310 033

## Description

### BACKGROUND

Revision total hip arthroplasty (THA) is a commonly used procedure for treating a patient that previously underwent a THA procedure but that requires further treatment. Specifically, over time, implants, plates and other components from a previous THA procedure may degrade or become loose within the bones of the patient. In these situations, a revision THA procedure is performed to replace and/or correct an alignment of the components from the previous THA procedure. One way a revision THA procedure is performed involves using a trochanteric grip plate and cabling system to compress and secure detached bone fragments to allow for bony material to heal and incorporate with adjunct bone and to the new implants used in the revision surgery. Hip abductors constantly pull on the greater trochanter and induce motion of the detached bone fragments underneath the grip plate relative to other portions of the bone, which slows or even arrests bone regeneration. Further, over time, the tensioned cables of the cabling system loosen due to slippage under tension or due to erosion of the soft tissue and bone. The emergence of these conditions result in the detached bone fragments not being able to properly heal after the revision THA procedure.

Thus, there is a need for improvements in greater trochanteric fixation during a revision THA procedure. Examples of implant tools are described in US 2023/310033 A1, US 5658349 A, US 2011/009973 A1, and US 2014/200619 A.

### BRIEF SUMMARY

According to the invention, there is provided a kit for providing fixation of a proximal portion of a femur as defined in appended claim 1. Optional, preferred features of embodiments are defined in the corresponding dependent claims.

### BRIEF DESCRIPTION OF THE DRAWINGS

The features, aspects, and advantages of the present disclosure will become better understood with regard to the following description, appended claims, and accompanying drawings in which:
FIG. 1A is a perspective view of a bone fixation system according to an embodiment of the present disclosure.
FIG. 1B is another perspective view of the bone fixation system of FIG. 1A.
FIG. 1C is a side view of the bone fixation system of FIG. 1A.
FIG. 1D is another side view of the bone fixation system of FIG. 1A.
FIG. 1E is a top view of the bone fixation system of FIG. 1A.
FIG. 2A is a perspective view of an implant of the bone fixation system of FIG. 1A.
FIG. 2B is a perspective view of an implant of a bone fixation system according to an embodiment of the present disclosure.
FIG. 3A is a top view of a bone plate of the bone fixation system of FIG. 1A.
FIG. 3B is a perspective view of the bone plate of FIG. 3A.
FIG. 3C is a bottom view of a proximal end of the bone plate of FIG. 3A.
FIG. 4A is a perspective view of a guide of the bone fixation system of FIG. 1A.
FIG. 4B is a cross-sectional view of the guide of FIG. 4A.
FIG. 4C is another cross-sectional view of the guide of FIG. 4A. FIG. 5A is a side view of the bone fixation system of FIG. 1A.
FIG. 5B is a side cross-sectional view of the bone fixation system of FIG. 1A.
FIG. 6A is a top cross-sectional view of the bone fixation system of FIG. 1A.
FIG. 6B is another top cross-sectional view of the bone fixation system of FIG. 1A.
FIG. 7A is side view of a bone plate locking bolt of the bone fixation system of FIG. 1A.
FIG. 7B is a side partial cross-sectional view of the bone fixation system.
FIGS. 8A-15B show steps in a method of using a bone fixation system.

### DETAILED DESCRIPTION

As used herein, the terms "about," "generally," and "substantially" are intended to mean that slight deviations from absolute are included within the scope of the term so modified.

As used herein, the term "proximal," when used in connection with a surgical tool or device, or components of a device, refers to the end of the device closer to the user of the device when the device is being used as intended. On the other hand, the term "distal," when used in connection with a surgical tool or device, or components of a device, refers to the end of the device farther away from the user when the device is being used as intended. However, when used in connection with the human body, the term "proximal" means closer to the heart, and the term "distal" means further from the heart. As used herein, the terms "substantially," "generally," "approximately," and "about" are intended to mean that slight deviations from absolute are included within the scope of the term so modified.

While the present disclosure provides exemplary applications of the contemplated system for a revision total hip arthroplasty (THA) procedure, it should be appreciated that the contemplated system may also be used in original THA procedures and in areas of the body other than the hip, such as the shoulder.

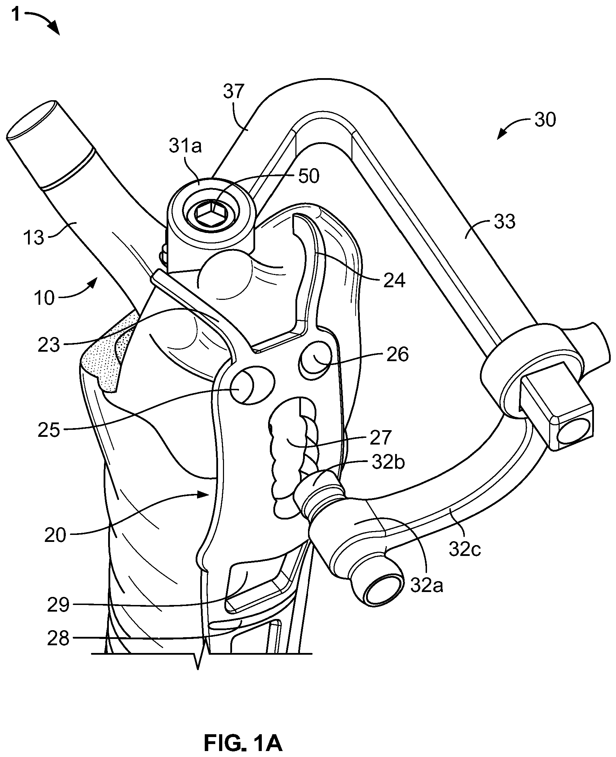

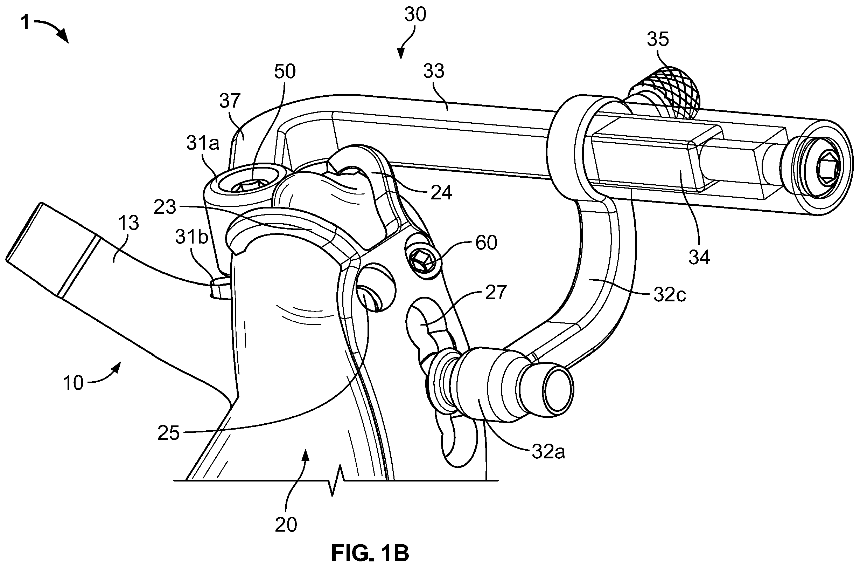

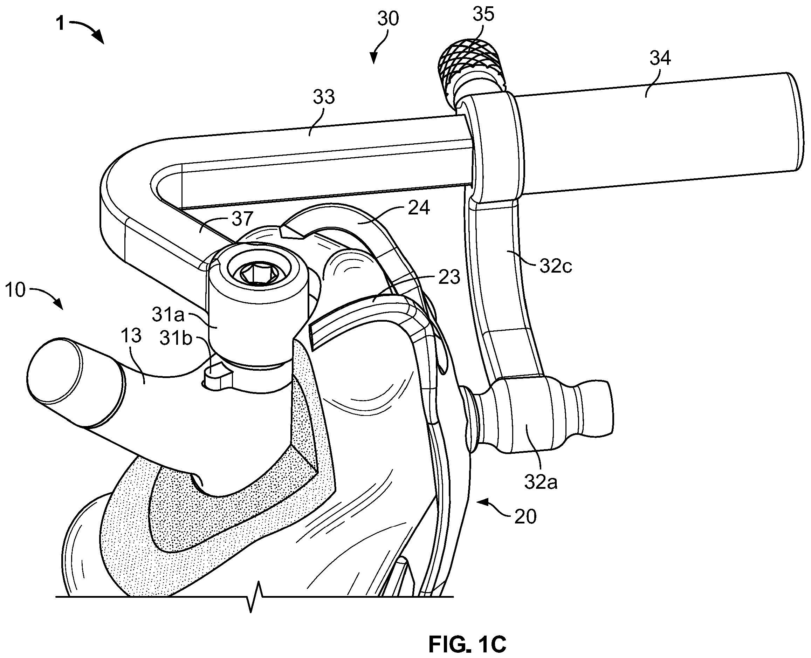

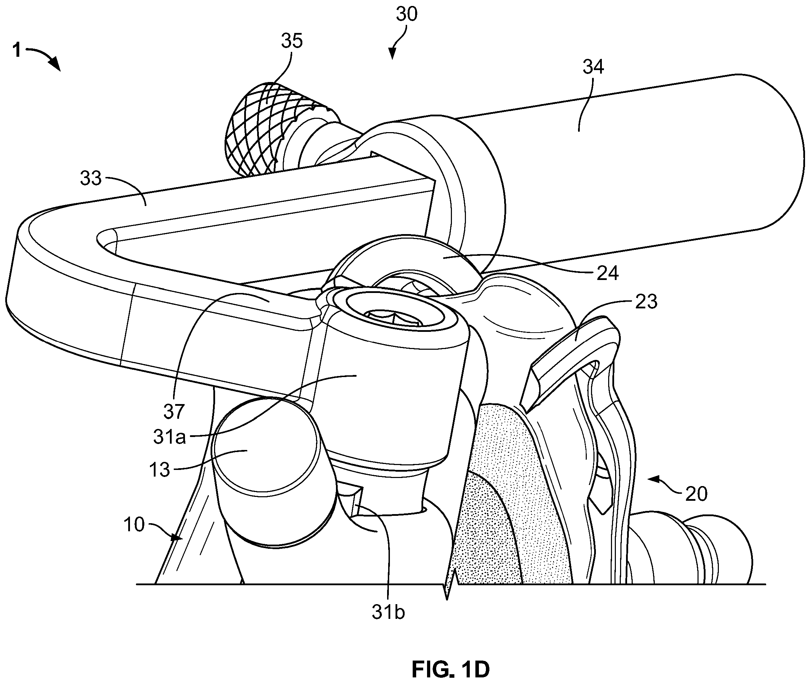

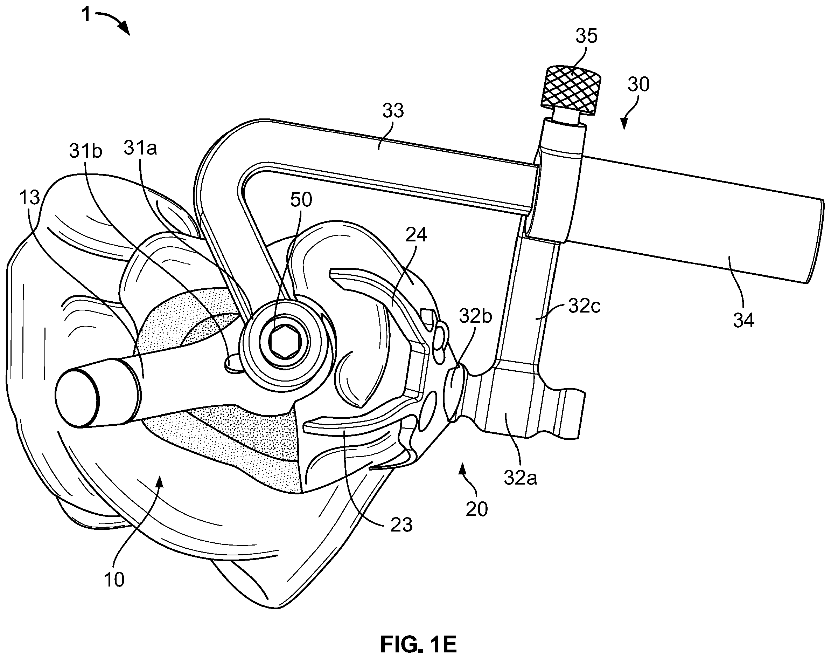

In one aspect, the present disclosure relates to an improved system for use in revision total hip arthroplasty (THA) procedure. FIGS. 1-7B illustrate a bone fixation system 1 according to one embodiment. In one example, system 1 is used to optimize bone healing during revision THA procedures by providing for the rigid greater trochanteric fixation. While system 1 is depicted and described for use in a femur of a patient, system 1 may be used in other long bones such as the tibia and fibula. System 1 includes a femoral implant 10, a bone plate 20 and a locking bolt 40. In some embodiments, the system 1 also includes a drill guide 30. In some variations of these embodiments, a system with or without a drill guide 30 may be included as part of a kit. Where the femoral implant includes a body and a separate stem, the system may also include a bolt 50 to connect the two components. Femoral implant 10, bone plate 20, locking bolt 40 and bolt 50 may be comprised of titanium alloy, cobalt chrome, or any other commonly used metal alloys. Additionally, the contemplated systems and kits may include one or more locking bone screws 60, one or more bands 70, or both for fixation of the bone plate 20 to the long bone. We now turn to the various components of the system. It should be appreciated that individual components of the system are also contemplated as individual standalone devices.

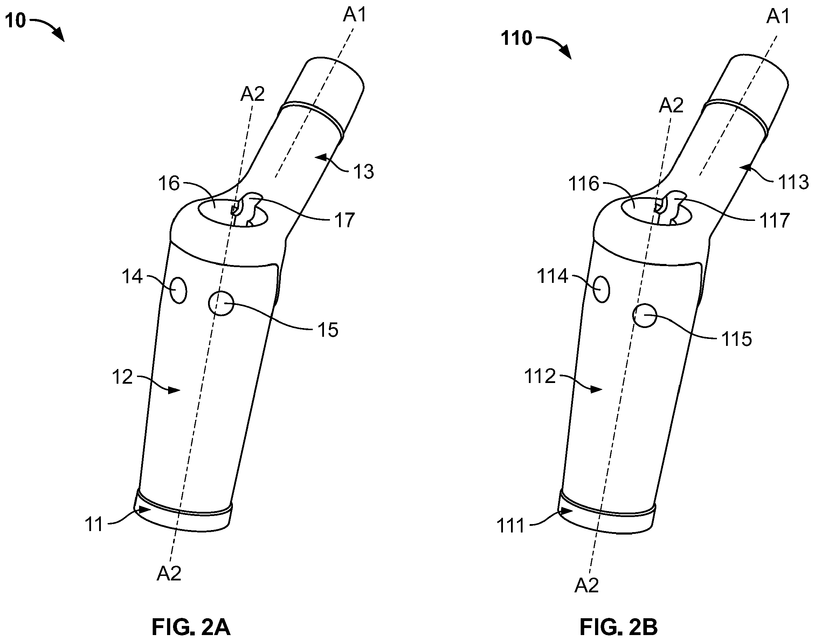

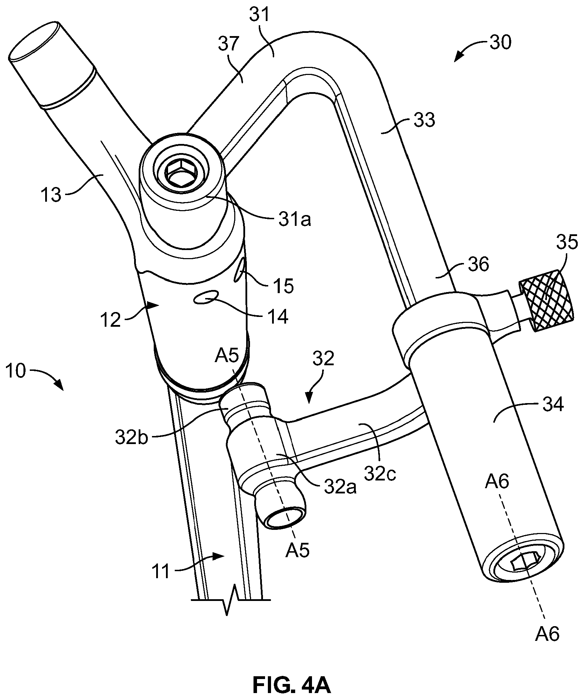

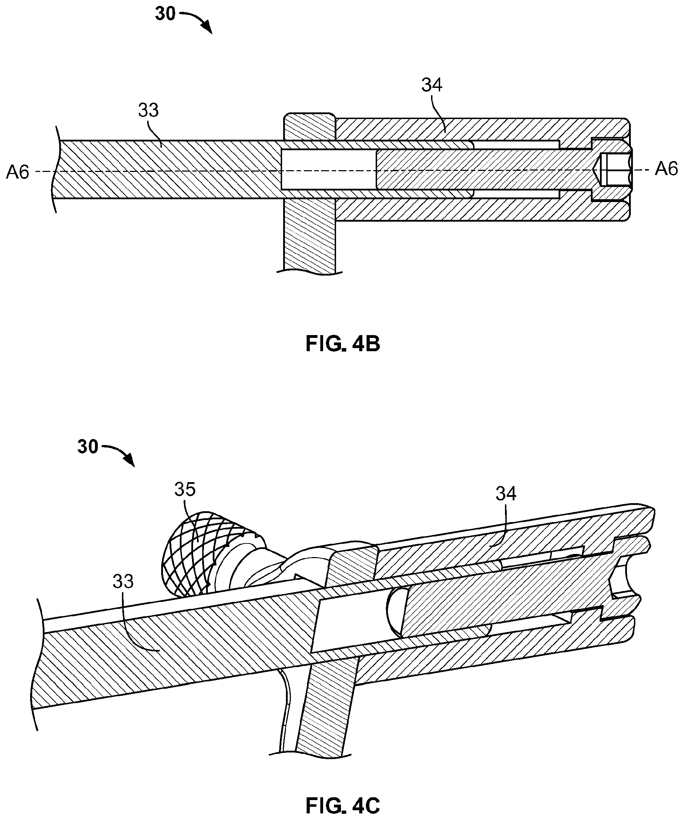

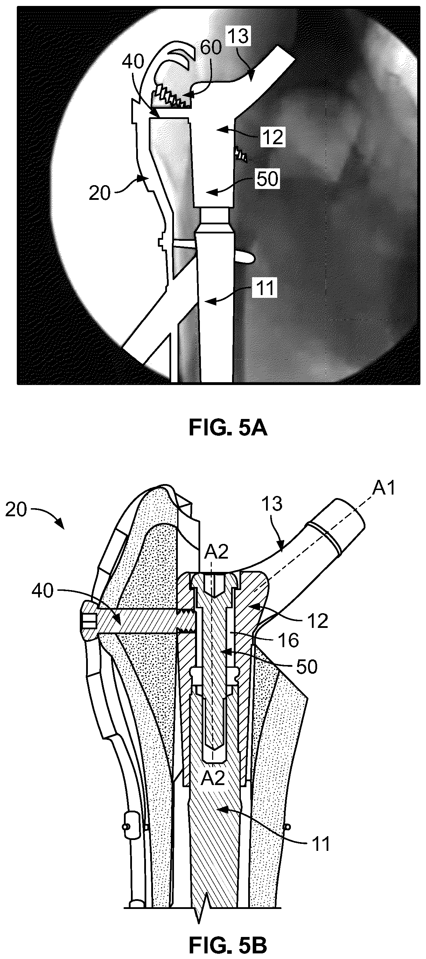

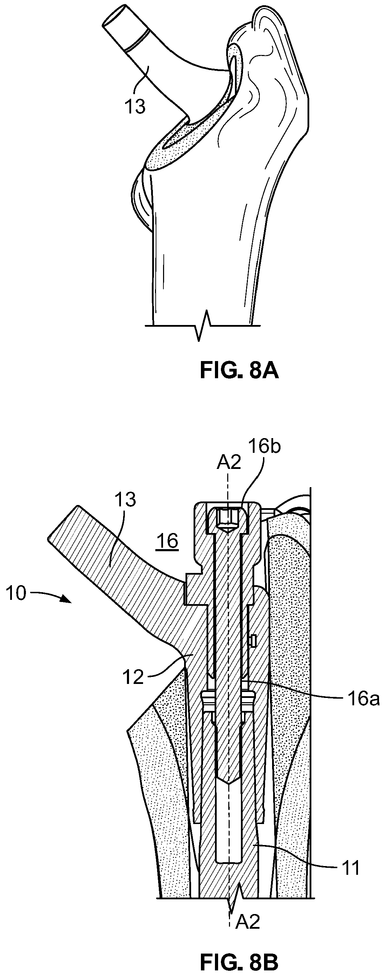

A femoral implant 10 of system 1 includes a femoral stem 11, a body 12 attached to the femoral stem 11, and a neck 13 extending from the body 12, as shown in FIGS. 2A and 5B. Femoral stem 11 of implant 10 is only partially shown in FIG. 2A, but can also be seen in FIGS. 4, 5B and 8B. In use, proximal body 12 is configured to be disposed over femoral stem 11 within the femur such that neck 13 extends outward from the femur so that when repositioned to the pelvis, the neck extends toward the acetabulum of the hip, as described further below. Proximal body 12 includes a first opening 14 and a second opening 15, each extending partially through a thickness of the proximal body. Openings 14, 15 are sized and otherwise configured to receive a locking bolt 40 used to fix a bone plate 20 to the outer surface of the femur, as described in detail further below. In one example, an interior surface of respective openings 14, 15 is threaded to receive a threaded end of locking bolt 40, the threads being complementary. As depicted in FIG. 2A, openings 14, 15 are located near the proximal end of proximal body 12 on a side of the proximal body 12 that is generally opposite where neck 13 extends away from the proximal body. In FIG. 2A, openings 14, 15 are located at the same depth on a length of the proximal body such that openings are at the same distance from the proximal end of proximal body. As depicted in FIG. 8B, channel 16 extends internally through proximal body 12 from the proximal end 16b to the distal end 16a and is configured to receive an implant locking bolt 50 to fix the proximal body 12 to distal stem 11, as described further below. Implant 10 is configured to be positioned in either a left femur or a right femur during a revision THA procedure.

With continued reference to implant 10, neck portion 13 of implant 10 extends from proximal body 12 along an axis A1, as shown in FIGS. 2A, 5B and 6B. A central longitudinal axis of each of the respective first and second openings 14, 15 of proximal body is transverse relative to axis A1 extending through proximal body 12 and neck portion 13. Further, a second plane coincident with axis A1 and a central longitudinal axis A2 of the proximal body 12, as shown in FIGS. 2A, 5B and 8B, is transverse to a first plane coincident with a central longitudinal axis of the first opening 14 and the central longitudinal axis of the proximal body and is also transverse to a third plane coincident with a central longitudinal axis of the second opening 15 and the central longitudinal axis of the proximal body. In the depicted embodiment, openings 14, 15 are offset from the second plane by an equal amount, but openings 14, 15 may be offset from axis A1 by differing amounts as required for the patient. By having each opening 14, 15 offset by an equal amount from the second plane, implant 10 is positionable in either the left or right femur of a patient while still being able to receive locking bolt 40 when it is passed through bone plate 20 and the femur. Further, in the depicted embodiment, openings 14, 15 are each angled relative to the second plane by approximately 25 degrees. Openings 14, 15 being oriented as shown in the depicted embodiment allows locking bolt 40 to extend through a thicker portion of the greater trochanter region of the femur which establishes are stronger and more fixed connection between bone plate 20 and proximal body 12 of implant 10. In other examples, openings 14, 15 may each be angled relative to the second plane by any amount within a range from 0 degrees to 30 degrees. In some of these examples, each opening may be angled a different amount relative to the second plane, while in other examples, each opening may be angled the same amount. In still further examples, implant 10 may include three or more openings.

In an alternative embodiment, the implant may be implant 110 shown in FIG. 2B, where like reference numerals refer to like elements of implant 10 in FIG. 2A, unless otherwise noted. In implant 110 of FIG. 2B, openings 114, 115 are located at different distances from the proximal end of proximal body such that first opening 114 is at a first distance from the proximal end of proximal body and the second opening 115 is at a second distance from the proximal end of proximal body. In this arrangement, the first distance is less than the second distance such that the first opening 114 is closer to the proximal end of proximal body 112 than second opening 115.

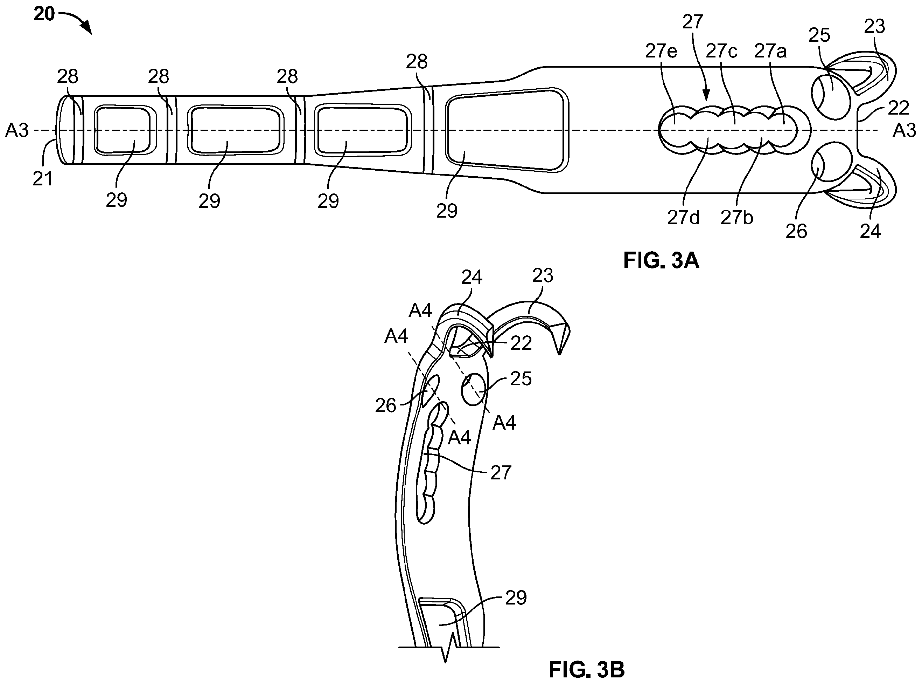

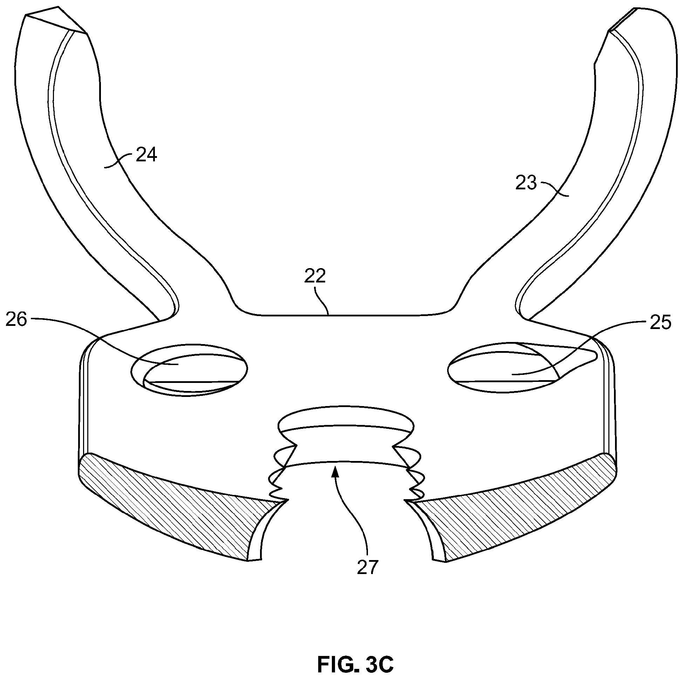

With continued reference to system 1, the system also includes a bone plate or grip plate 20, as best shown in FIGS. 3A- 3C. Bone plate 20 is configured to be positioned on either the left or right femur during a revision THA procedure. Bone plate 20 has a length extending from a distal end 21 to a proximal end 22 opposite the distal end. A first prong 23 and a second prong 24 extend from the proximal end 22 in an outward direction from bone plate 20. In the depicted embodiment, prongs 23, 24 are configured to be positioned over the greater trochanter region of the femur to aid in the securement of bone plate 20 to an outer surface of the femur. Additionally, prongs 23, 24 extend slightly outward from a central longitudinal axis along the length of bone plate 20 as they extend away from proximal end 22 to provide increased coverage of the anterior posterolateral region of the femur when bone plate 20 is fixed to the femur. Although first and second prongs 23, 24 are shown on bone plate 20 in the depicted embodiment, any number of prongs may extend from bone plate 20. For example, a bone plate may include a single prong extending from the proximal end or three or more prongs. Prongs 23, 24 may also be shaped or modified in a variety of ways to complement a femur or other bone to be fixed by the bone plate 20. Additionally, proximal end 22 of bone plate 20 increases in thickness from the outer, i.e., lateral edges of bone plate towards the central axis A3 of bone plate adjacent an elongate opening 27, as best shown in FIG. 3C. In other words, the thickness of proximal end 22 of bone plate 20 is thickest proximal the center of bone plate, and thinnest at either outer edge of bone plate. The gradual increase in thickness across proximal end 22 of bone plate 20 allows increased fixation of the bone plate 20 over the femur by providing enhanced matching of the contours of the outer surface of the femur.

Within a body of bone plate 20, the bone plate includes a series of openings. In the depicted embodiment, these openings include holes 25, 26, elongate opening 27 and additional spaced apart openings 29 in a distal region of the bone plate. Elongate opening 27 is located adjacent proximal end 22 as shown in FIG. 3A, separated from proximal end 22 only by holes 25, 26. Elongate opening 27 has an elongated shape extending along the length direction of bone plate 20. Elongate opening 27 is defined by a series of circular cut-outs abutting each other along its length in the form of a plurality of receiving portions 27a, 27b, 27c, 27d, 27e, each designed to receive a partially spherical tip 32b of a guide 30, described in greater detail below. Each receiving portion of the plurality of receiving portions 27a, 27b, 27c, 27d, 27e is defined by a concave edge on an outer surface of the bone plate. In some examples, the inner surface of each of the plurality of receiving portions 27a-e may have a generally spherical contour to complement receipt of an object with a spherical surface, such as partially spherical tip 32b. Each receiving portion is overlapping with an adjacent receiving portion as shown in FIGS. 3A and 3B such that a circle defined by a radius at the surface of one receiving portion overlaps with a circle defined by a radius at the surface of an adjacent receiving portion. As depicted, each receiving portion has the same shape and diameter. Thus, the plurality of receiving portions 27a-e are interconnected such that an instrument end, e.g., the spherical tip of guide 30, is receivable in any one of the receiving portions as deemed appropriate for a particular patient. While the depicted embodiment shows only receiving portions 27a-e, any number of receiving portions may be included to define elongate opening 27 for bone plate 20. Additionally, elongate opening 27 and the respective receiving portions 27a-e may be shaped for receipt of a bone locking bolt 40 therethrough for fixation of the bone plate 20 to the proximal body 12 of implant 10.

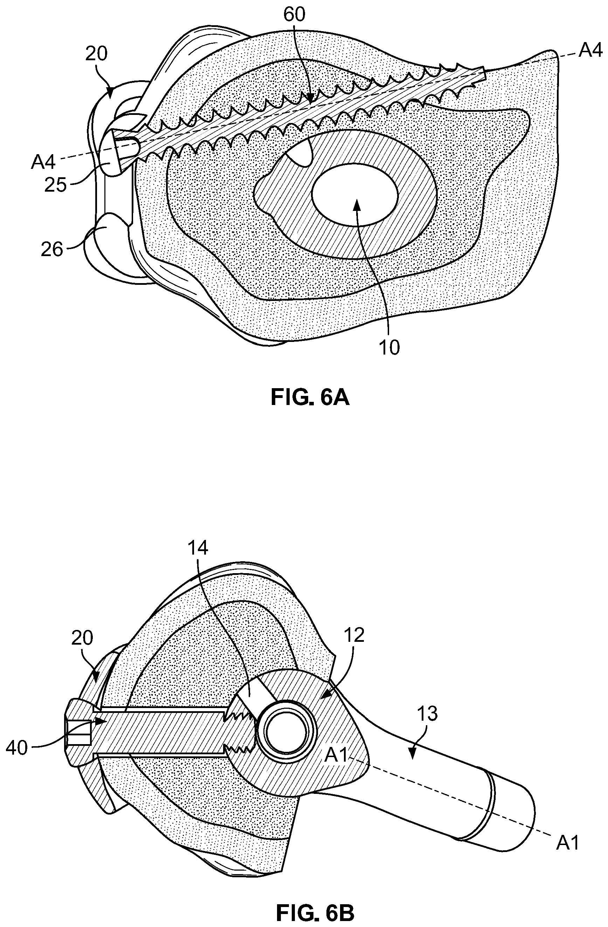

Also on the body of bone plate 20 are first hole 25 and second hole 26. First and second holes 25, 26 are immediately adjacent proximal end 22 and are located between proximal end 22 and elongate opening 27. As depicted, holes 25, 26 are generally circular in shape at an upper surface of the bone plate and each is configured to receive locking bone screws 60 for fixing bone plate 20 to the femur, as described further below. Each of holes 25, 26 are approximately the same distance from proximal end 22 and are on opposite sides of a central longitudinal axis A3 of bone plate 20. Each hole 25, 26 is oriented such that a central axis of the hole extends further away from the central longitudinal axis A3 of bone plate 20 from an upper surface of the bone plate to the bone-facing surface of the bone plate. The angulation of the holes 25, 26 is shown in FIGS. 3A, 3B and 6A. A central longitudinal axis A4 extends through each of holes 25, 26 that is at an acute angle relative to the bone-facing surface and the central longitudinal axis A3 of the bone plate 20. Although first and second holes 25, 26 are shown in the depicted embodiment, any number of holes can be included in the bone plate to fix bone plate 20 to the femur with a desired number of locking bone screws 60. Further, variations may include holes at different locations along the length of the bone plate and at different offsets relative to the central longitudinal axis of the plate. Further, the holes may have different shapes and/or angles relative to the depicted embodiment and in some examples, the bone plate may have two or more holes that are different from each other.

Bone plate 20 further includes a plurality of grooves 28, each groove of the plurality of grooves extending across the width of the bone plate and being spaced apart from the other grooves along the length of the bone plate. Each groove 28 is an indentation on an upper surface of bone plate 20 and is configured for receiving a cable or band 70 therein to hold and compress bone plate 20 against the femur, as described further below. In the depicted embodiment, grooves 28 are disposed in the distal region of the bone plate 20. However, grooves 28 may be disposed along any portion of bone plate 20 in order to receive the desired bands 70 for fixation of the plate to bone.

Optionally, and in the depicted embodiment, bone plate 20 further includes a plurality of spaced apart openings 29 in the distal region of the bone plate. Each of the spaced apart openings 29 extend through an upper surface of the bone plate 20 to the bone-facing surface of the bone plate. Each of the spaced apart openings may have a width slightly less than a width of the bone plate. Further, each of the spaced apart openings 29 are separated from one another by a cross-portion of plate that includes a groove 28, as shown in FIG. 3A. In use, spaced apart openings 29 provide aid in the visualization of the femur and bodily tissue. Additionally, spaced apart openings 29 reduce the overall weight of bone plate 20.

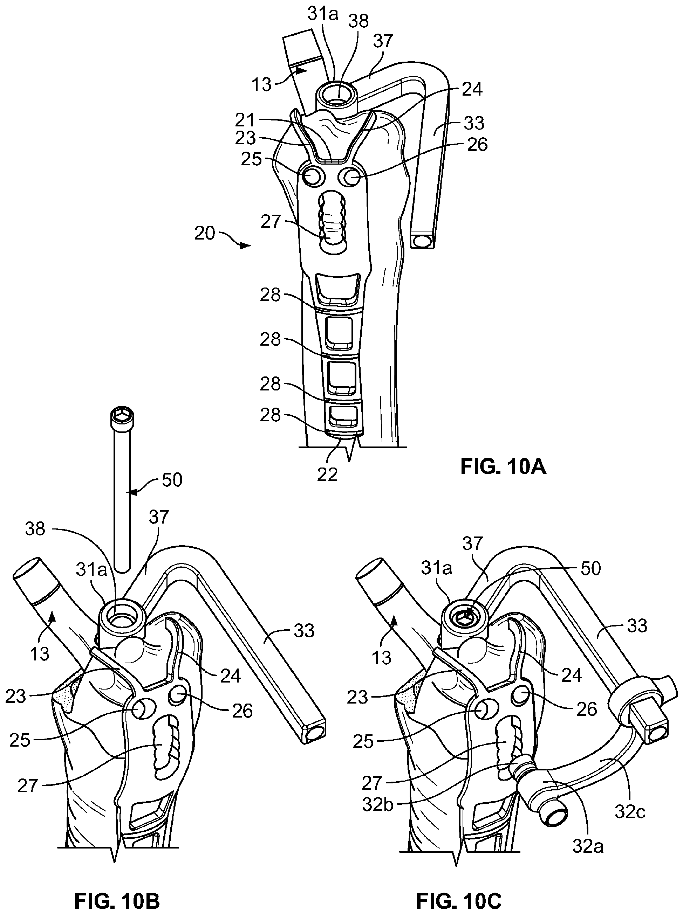

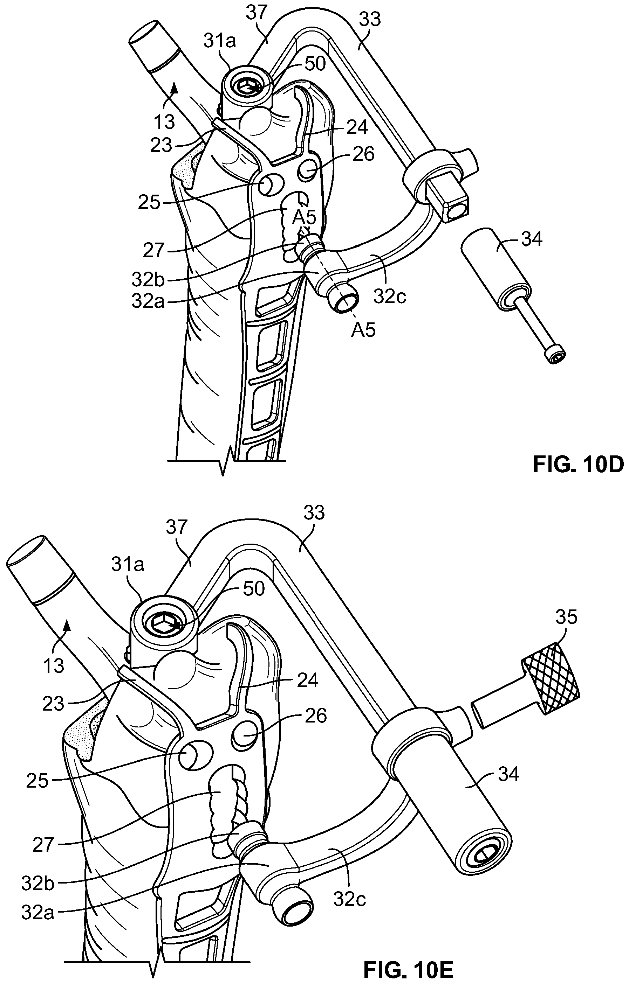

Targeting guide 30 of system 1 is shown in FIG. 4A as attached to implant 10. Guide 30 includes a first end arm 31, a spacer arm 33 extending from the first arm, a second end arm 32 slidably attached to the spacer arm 33, and an engagement shaft 34 slidably attached to the spacer arm and in contact with second end arm. First arm 31 includes an attachment portion 31a configured for attachment to the proximal body 12 of implant 10. Additionally, attachment portion 31a includes an attachment tab 31b extending outward from the attachment portion configured for attachment to the implant 10. Attachment tab 31b, as best shown in FIGS. 1B-1E, is configured to be received within an attachment slot 17 of implant 10 between neck 13 and proximal body 12. A central axis extending though attachment tab 31b is aligned with axis A1 extending though neck 13, as best shown in FIGS. 2A-2B and FIG. 6B. Thus, alignment tab 31 is designed to align either one of first or second openings 14, 15 of proximal body 12 of implant 10 with a central axis of the free end shaft 32a of guide 30 along axis A5, as discussed further below. Spacer arm 33 includes a shaft extending at an angle from first arm 31, as shown in FIG. 4A, to a free end, as shown in FIGS. 10A-10D. Free end of spacer arm 33 includes a threaded hole configured to receive a locking screw for fixation of engagement shaft 34 to spacer arm 33, as best shown in FIGS. 4B-4C. In one embodiment, engagement shaft 34 may include a square or cuboid lumen to receive free end of spacer arm 33, and a free end of engagement shaft 34 may include an opening for receiving the locking screw to fix engagement shaft to spacer arm 33. Spacer arm 33 further includes a spacer marking 36 disposed near the free end of the spacer arm. Spacer marking 36 may indicate a size of the locking bolt 40 necessary for securing bone plate 20 to implant 10, as discussed further below. Second end arm 32 includes, transverse extension arm 32c and cannulated free end shaft 32a, where a first end of the transverse extension arm includes a loop portion slidable over spacer arm 33 and a second end of transverse extension arm is attached to free end shaft 32a. Free end shaft 32a includes an elongate dimension aligned with the cannulation of the free end shaft, and at one end of the shaft includes a partially spherical tip 32b. Second end arm 32 is slidably engageable to spacer arm 33 via sliding of the loop portion of second end arm 32 over spacer arm 33, as shown in FIG. 4A. When second end arm 32 is engaged to spacer arm 33, partially spherical tip 32b faces attachment portion 31a of first end arm, also shown in FIG. 4A. Engagement between second end arm 32 and spacer 33 is adjustable. Specifically, second end arm 32 includes a transverse opening adjacent the loop portion sized to receive a securement member such as knob 35. Knob 35 is rotatable to control whether second end arm 32 is locked relative to spacer arm 33 by controlling whether a tip of a shaft extending from knob (not shown) applies sufficient pressure to spacer arm 33 to prevent movement of second end arm 32. When second arm 32 is unlocked relative to spacer arm 33, second end arm 32 is slidable in either direction along an axis A6, as shown in FIG. 4A.

Similar to implant 10 and bone plate 20, guide 30 is configured for use with either the left or right femur during a revision THA procedure. Guide 30 is modular such that spacer arm 33 is interchangeable with all other components of guide, and spacer arm 33 has a left and a right embodiment to properly position free end shaft 32a in alignment with first or second opening 14, 15 of proximal body. For example, in the depicted embodiment, spacer arm 33 is shown in a configuration for a left femur as evidence by a configuration marking 37 represented by the letter "L". In this configuration, spacer arm 33 is configured to position free end shaft 32a in alignment with proximal body 12 of implant 10 that is positioned within a left femur of the patient. Additionally, alignment tab 31b is positioned within alignment slot 17. In this example, implant 10 is positioned in the left femur of the patient. In this position, first opening 14 of proximal body 12 of implant 10 is in alignment with a central axis of the free end shaft 32a of guide 30. In other words, in this configuration of system 1, axis A5 extends through both a central position of the opening of free end shaft 32a of guide 30 and a central position of first opening 14 of proximal body 12. Free end shaft 32a is then configured to guide a drill through the bone or a bone plate locking bolt 40 through the femur along axis A5 and into the first opening 14 of the proximal body 12. While the above describes alternatives where spacer arm 33 may be substituted for left and right variations, it should also be appreciated that second end arm 32 may be substituted with other second end arms having different offsets between free end shaft 32a and the first end of transverse extension arm 32c, different sized cannulations or end tips in free end shaft 32a, different sized lengths of spacer arm 33, and different types of attachment portions 31a for fixing guide 30 to implant 10.

As mentioned above, while the depicted embodiment is configured for placement in a left femur, guide 30 may also be modified for use in a right femur. For example, spacer arm 33 also includes a right embodiment to be used with a right femur, and which may be evidenced by a configuration marking 37 represented by the letter "R". In this example, a guide 30 configured for placement in a right femur may have a spacer arm 33 configured to connect to the implant 10 in the same manner as discussed above, but spacer arm 33 will extend from implant in opposite direction from the guide 30 depicted embodiment in FIG. 4A. Additionally, alignment tab 31b is similarly positioned within alignment slot 17. In this way, the right embodiment of spacer arm 33 will position free end shaft 32a of guide 30 with a second opening 15 of proximal body 12 of implant 10. In other words, in this configuration of system 1, axis A5 extends through both a central position of the opening of free end shaft 32a of guide 30 and a central position of second opening 15 of proximal body 12.

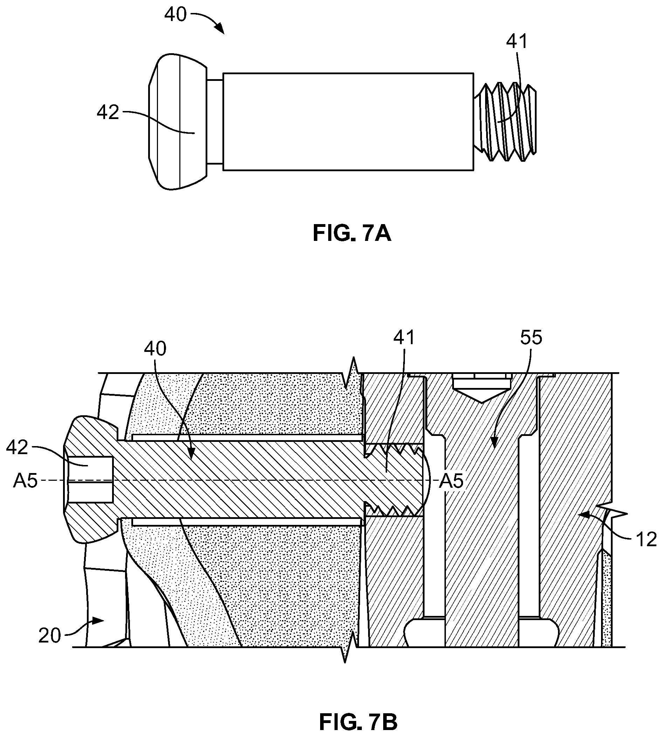

With continued reference to individual components that may be included in system 1, a bone plate locking bolt 40 is shown in FIGS. 7A-7B. As described in greater detail below, locking bolt 40 may be used to secure bone plate 20 to implant 10. Locking bolt 40 includes a threaded leading end 41 extending from an elongated shaft of the locking bolt and a head 42 extending from the elongated shaft opposite threaded leading end 41. Locking bolt 40 may include a drive in the head for receipt of a tool to facilitate threaded engagement of the locking bolt with an implant. Variations of locking bolt 40 may include any fixation feature configured to fix a bone plate to an implant through a bone.

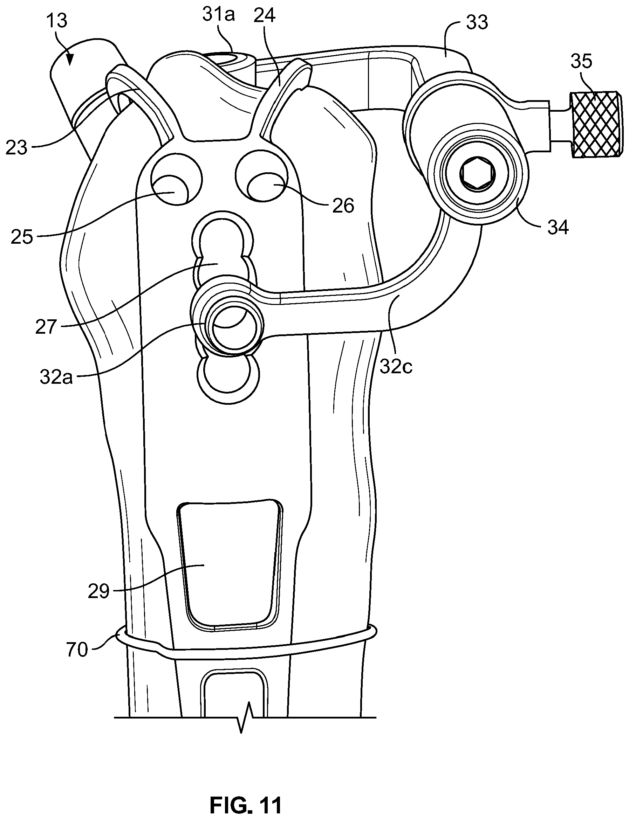

Another component that may be included in system 1 is implant locking bolt 50 as shown in FIG. 5B. Locking bolt 50 comprises an elongated shaft extending from a head portion for fixing a proximal body 12 and distal body 11 of a femoral implant 10 to each other. Further, yet another component that may be included in system 1 is a locking bone screw 60, as shown in FIGS. 5A and 6A. Locking bone screw 60 includes a threaded shaft extending from a head portion for fixation within a bone. A flexible band 70 as shown in FIGS. 11-15B is yet another component that may be included in system 1. Such band is formed of materials having properties sufficient to hold bone plate 20 against a bone surface.

Turning now to the arrangement of the system when the various components are assembled, FIG. 5A depicts system 1 in an assembled state in a patient. Specifically, the implant 10 is positioned within a proximal portion of a femur and the bone plate 20 positioned on an outer surface of the femur. As implanted, implant 10 includes body 12 received on stem 11. An implant locking bolt 50 fixes the proximal body 12 to stem 11. Implant locking bolt 50 is positioned within implant 10 along a longitudinal axis extending through a lengthwise direction of the femur and within the internal channel 16 of implant, as shown in FIG. 5B.

In a fully assembled system 1, bone plate 20 is secured to implant 10. Securement of these components may be through locking bolt 40. Specifically, locking bolt 40, as shown in FIGS. 5B, 6B and 7B, for example, is disposed through a suitable location within elongate opening 27a-e of bone plate 20 and into one of openings 14, 15 of implant 10. In this fixed state, locking bolt 40 extends through bone plate 20, through part of the femur, and into an opening in proximal body 12, all along axis A5. A threaded leading end 41 of locking bolt 40 threadably engages the first or second opening 14, 15 of the proximal body 12 to hold bolt 40 relative to implant and a head 42 of locking bolt abuts an outer surface of bone plate 20 to prevent detachment of bone plate 20 from bolt 40 or the bone surface. In the depicted embodiment, as shown in FIG. 6B, the leading end 41 of locking bolt 40 threadably engages second opening 15 of proximal body 12. Thus, locking bolt 40 secures bone plate 20 against the outer surface of the femur and to proximal body 12 positioned within the femur.

Bone plate 20 is further fixed to femur by one or more locking bone screws 60, with a single bone locking screw 60 shown in FIGS. 5A and FIGS. 6A. With continued reference to the depicted embodiment, locking bone screw 60 is disposed through first hole 25 of bone plate 20 into the femur of the patient, reaching cortical bone on an opposite side of the bone from an entry location into the bone. While only a single locking bone screw 60 is shown in the depicted embodiment, a second locking bone screw may be disposed through the second hole 26 of bone plate 20 into the femur in a similar manner. Directed by the shape of hole 25, locking bone screw 60 extends through first hole 25 along axis A4 in an inferior direction into the femur as shown in FIG. 5A. In some example implantations, positioning of locking bone screw 60 in such manner results in the locking bone screw 60 extending through cortical bone of the greater trochanter of the femur, through cancellous bone of the femur, and through the cortical bone of the lesser trochanter of the femur. This trajectory of locking bone screw 60 provides the benefit of a stronger fixation of bone plate 20 to the femur as a distal tip of locking bone screw 60 is fixed to harder cortical bone in the lesser trochanter rather than softer cancellous bone within an interior of femur. As depicted, locking bone screw 60 extends through the femur adjacent a first side of implant 10. In other examples, a second locking bone screw 60 extends through second hole 26 and into the femur in a similar manner as described above for the first locking bone screw 60, but the second locking bone screw extends through the femur adjacent a second side opposite first side of implant 10 and its tip extends into cortical bone on that second side.

In another aspect, the present application relates to a kit including a combination of components including at least one component of the system. In some embodiments, a kit may include one or more components from system 1. For example, the kit may include an implant 10, bone plate 20, guide 30, one or more bone plate locking bolts 40, one or more implant locking bolts 50, one or more locking bone screws 60 and/or one or more bands 70. In some examples, the kit may further include a plurality of engagement shafts 33 for use with guide 30 or a plurality of second end arms 32 for use with guide 30. In still further examples, a kit may include implant 10 and bone plate 20, one or more bone plate locking bolts 40, one or more implant locking bolts 50, one or more locking bone screws 60 and one or more bands 70. In still further examples, the above examples of a kit may include no bolts, screws or bands or only some of the aforementioned bolts, screws and bands.

Any combination of system components may also be included in a single package or in individual packages which may later be brought together to create a kit. It is also contemplated that a kit may include any combination of system components along with one or more additional instruments used to place such securement devices in a patient. In other examples, the kits contemplated herein may be accompanied by an instruction manual on how to perform one or more of the methods of using the contents of the kit.

In another aspect, the present disclosure not forming part of the claimed invention relates to a method for performing a surgical procedure for repairing a bone fracture on a femur, and specifically a revision total hip arthroplasty (THA) procedure. As explained elsewhere in the present application, it should be appreciated that the system and components thereof as contemplated by the present disclosure are not limited to use in THA procedures. And, to the extent the methods below are described with respect to a THA procedure, such description is for purposes of illustration. It should be understood that the following operations do not have to be performed in the exact order described below. Instead, various steps may be handled in a different order or simultaneously. Steps may also be omitted or added unless otherwise stated herein.

In one example not forming part of the claimed invention, a method for repairing a bone fracture begins with a first step where an implant 10 is positioned into an intramedullary canal of a femur of a patient by a generally accepted method or another known method. As previously discussed, implant 10 is configured for surgical procedures in either the right or left proximal femur. In one non-limiting example, implant 10 is positioned within a proximal portion of a left femur of the patient, as best shown in FIGS. 8A-8B. In this position, a femoral stem 11 of the implant 10 is positioned within an intramedullary canal of the femur. Additionally, a proximal body 12 is disposed over stem 11 such that a femoral neck portion 13 partially extends from proximal body 12 for expected receipt in an acetabulum of the pelvis.

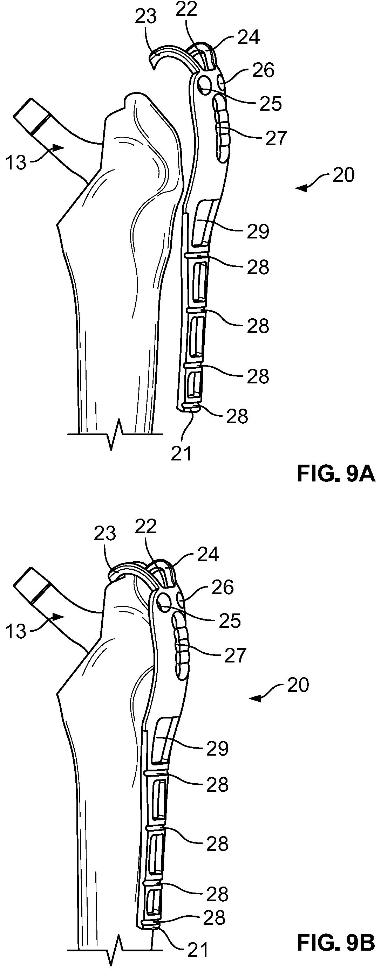

In a second step, a bone plate 20 is positioned on an outer surface of the femur, as best shown in FIGS. 9A-9B. Bone plate 20 is positioned along the bone fracture and detached bone fragments such that prongs 23, 24 and a proximal end 22 of bone plate are positioned over a greater trochanteric region of the femur, while distal end 21 of bone plate 20 is positioned over the femoral shaft of the femur.

In a third step, a targeting system or guide 30 is fixed to proximal body 12 of implant 10 and positioned over bone plate 20, as best shown in FIGS. 10A-10C. In the depicted embodiment, guide 30 is configured for use with the implant 10 positioned within the left femur of the patient, as shown by a configuration marking 37 proximal to an attachment portion 31a of the guide. Targeting guide 30 is fixed to the implant 10 by a locking bolt or screw that extends through an opening 38 of the attachment portion 31a of the first end arm 31 of guide 30 into the channel 16 of the implant. After fixing guide 30 to implant 10, second end arm 32 is engaged with spacer arm 33. Specifically, second end arm 32 is advanced over spacer arm 33 until spherical tip 32b of free end shaft 32a is positioned over an opening of the elongate opening 27a-e of bone plate 20. In some examples of the method, engagement shaft 34 is used to push second end arm 32 along spacer arm 33. Spherical tip 32b of free end shaft 32a of second arm 32 has a shape that is receivable by at least one of receiving portions 27a-e of bone plate 20. In the depicted embodiment, spherical tip 32b of free end shaft 32a is spherical in shape and is configured to be press-fit into any one of receiving portions 27a-e. In use, free end shaft 32a is positioned in one of receiving portions 27a-e and presses against bone plate 20 in a direction along axis A5 against the outer surface of the femur in a direction towards proximal body 12 of implant 10. Stated another way, such pressure by second end arm 32 against bone plate 20 compresses bone plate 20 against the femur.

In a fourth step, guide 30 compresses bone plate 20 against the femur, as best shown in FIG. 10E. Spherical tip 32b of free end shaft 32a is positioned within one of the receiving portions 27a-eof elongate opening 27 so that its position provides a desired trajectory for insertion of bone plate locking bolt 40, as described further below. After positioning spherical tip 32b of free end shaft 32a within the desired fixation portion 27a, 27b, 27c, 27d, 27e, an engagement shaft 34 is assembled to guide 30 and moved in a direction towards the femur along axis A6 in order to compress bone plate against the outer surface of the femur. The compression bone plate 20 allows for self-alignment of the bone plate relative to the implant 10 within the femur. This provides the benefit of assuring proper alignment of elongate opening 27 of bone plate 20 relative to one of the first or second opening 14, 15 of implant 10 prior to performing any resection or drilling of the femur, as described further below.

In a fifth step, the compressed bone plate 20 is further secured to femur by a cable or band 70, as best shown in FIG. 11. Band 70 is disposed within a groove 28 of bone plate 20 and is tied into a closed loop that extends around and encloses the femoral shaft of the femur. In the depicted embodiment, band 70 is disposed in a groove 28 which is positioned distally from the opening 29 and a proximal portion of bone plate 20. While only a single band 70 is shown disposed within a single groove 28 of the depicted embodiment, a plurality of grooves may receive a respective plurality of bands, respectively. Thus, proximal end 22 of bone plate 20 is compressed against the femur by spherical tip 32b of free end shaft 32a of guide 30, and the shaft and distal end 21 of bone plate 20 is compressed against the femur by at least one band 70.

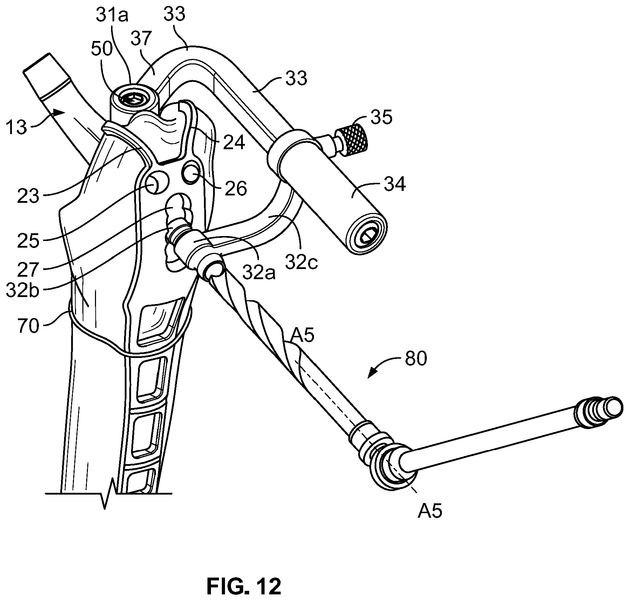

In sixth step, a bone reamer or drill 80 is used in conjunction with guide 30 in order to create a hole within the femur for receiving bone locking bolt 40, as best shown in FIG. 12. As guide 30 continues to compress bone plate 20 against the femur, drill 80 is positioned through the cannulation of free end shaft 32a of guide 30 and through elongate opening 27 of bone plate 20 against femur. Drill 80 is then operated to advance the drill bit into the femur along axis A5 and creates a hole extending through the femur to implant 10. Due to the self-alignment aspect of bone plate 20 from compression of guide 30, the drill 80 creates a hole extending from elongate opening 27 of bone plate to the first opening 14 of implant 10 such that the drilled hole is aligned with the first opening 14. In non-limiting examples, the hole created by drill 80 may have a diameter in a range from 5.0 mm to 8.0 millimeters.

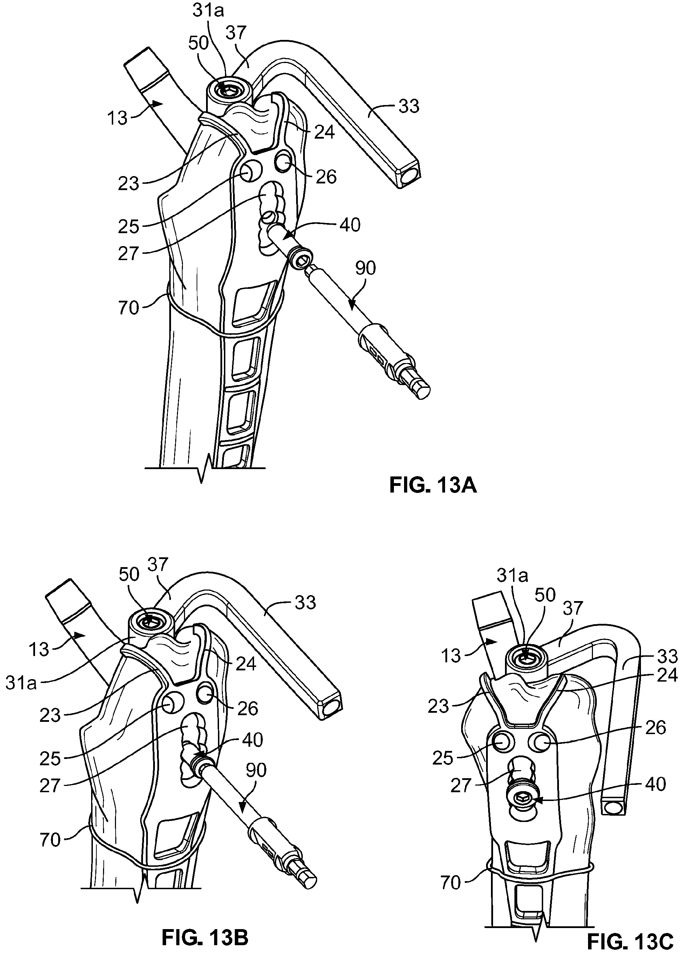

In seventh step, locking bolt 40 is used to fix bone plate 20 to implant 10 through the hole in the femur created by drill 80, as best shown in FIGS. 13A-13C. A size of locking bolt 40 corresponds to the spacer marking 36 identified on the spacer arm 33 relative to the position of second end arm 32. Prior to inserting bone locking bolt 40 into the femur, second arm 32 and spacer arm 34 of guide 30 are removed from the system 1. Bone locking bolt 40 is positioned within the hole created by drill 80, and bone locking bolt is advanced through the femur and driven into opening 14 of implant 10 by a locking driver 90. In the depicted embodiment, locking bolt 40 is threadably engaged with implant 10. In an implanted state, bone locking bolt 40 extends from elongate opening 27 of bone plate 20 through the femur and into opening 14 of implant 10, as best shown in FIG. 5B. A distal end of bone locking bolt 40 is threaded in order to create a threaded connection with opening 14 of implant 10. A proximal end of bone locking bolt 40 is configured to be fixed within a receiving portions 27a-eof elongate opening 27 to establish a connection and fixation of bone plate 20 to implant 10.

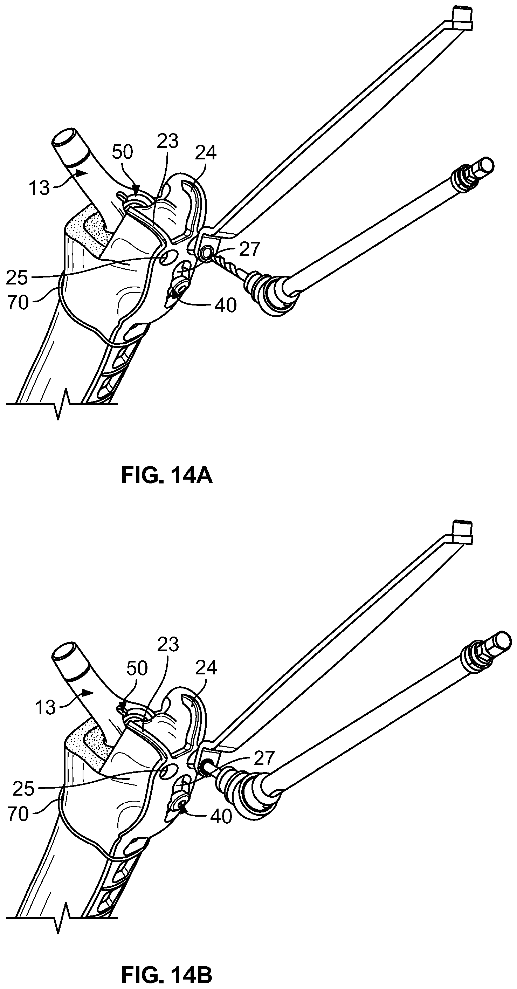

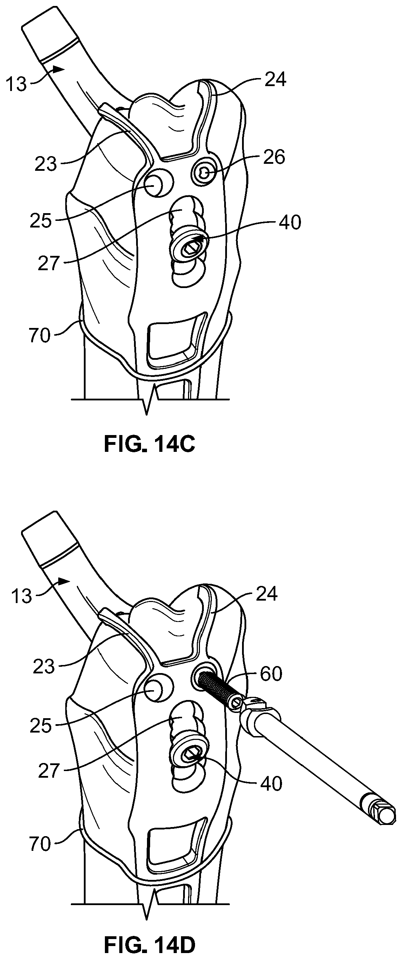

In an eighth step, a locking bone screw 60 is drilled into the femur through second hole 26 of bone plate 20, as shown in FIGS. 14C-14D. In the depicted embodiment, implant 10 is positioned within the proximal portion of the left femur of the patient. Therefore, locking bone screw 60 is drilled into the left femur through hole 26 of bone plate 20 and is positioned adjacent implant 10. In another example, however, the implant 10 is positioned within the proximal portion of the right femur of the patient, as previously discussed. In this example, a locking bone screw 60 is drilled into the femur through first hole 25 of bone plate 20 and is positioned adjacent implant 10 in the right femur. Continuing with the depicted embodiment, prior to inserting locking bone screw 60 into the femur, free end shaft 32a of guide 30 is disconnected from implant 10 and all of the remaining components of guide 30 are removed from the system 1. Additionally, prior to inserting locking bone screw 60 into the femur, a pilot hole may optionally be drilled through hole 26 of bone plate and into the femur, as best shown in FIGS. 14A-14B. Pilot hole for hole 25 26, however, may be optional depending on the type of locking bone screw 60 used. As previously discussed, bone screw 60 is fixed within the femur along an angled trajectory, i.e., along axis A4, adjacent implant 10. Additionally, in variations of the method, a first and second locking bone screw may be disposed into each of the bone plate holes.

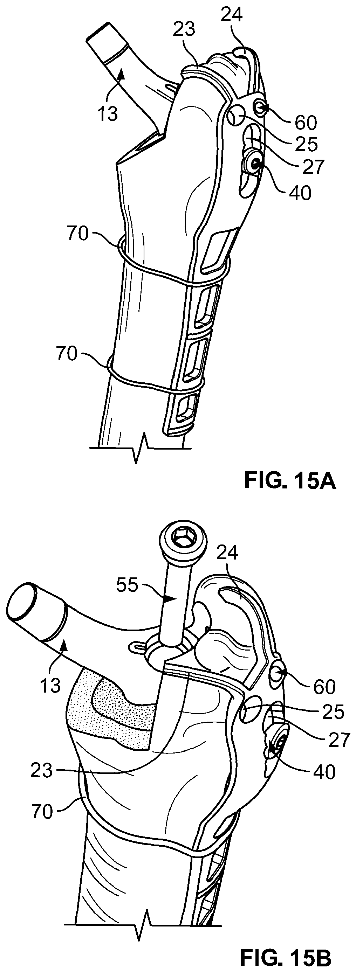

In a ninth step, with guide 30 removed from implant, an implant fixing bolt 55 is reinserted through channel 16 of implant 10 to fix the proximal body 12 to the stem 11, as best shown in FIGS. 15A-15B.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised within the scope of the present invention as defined by the appended claims.

## Claims

1. A kit for providing fixation of a proximal portion of a femur, the kit comprising:
a locking bolt (40);
an implant (10, 110) including a stem (12, 112) and a neck (13, 113) extending from the stem, the neck having an elongate dimension extending along a first axis (A1), the implant being configured to be disposed within the proximal portion of the femur,
wherein the stem includes a stem opening (14, 15; 114, 115) for receipt of the locking bolt, the stem opening being centered along a second axis (A5) transverse to the first axis, and
wherein the first axis extends through a first plane, the first plane being defined by a first end of the stem, a second end of the stem, and the first axis; and
a bone plate (20) configured to engage an outer surface of the proximal portion of the femur, the bone plate including a bone plate opening (27) for receipt of the locking bolt,
wherein the locking bolt is configured to pass through the bone plate opening and into the stem opening to secure the bone plate to the implant,
the kit being **characterized in that** the second axis is angled relative to the first axis by a range of 5 to 30 degrees.

2. The kit of claim 1, wherein the stem opening (14, 15; 114, 115) includes a first stem opening and a second stem opening and a third axis traverse to the first axis extends through the first stem opening and a fourth axis traverse to the first axis extends through the second stem opening.

3. The kit of claim 2, wherein the third axis is angled relative to the first axis and the fourth axis is angled relative to the first axis by an equal degree.

4. The kit of claim 2 or 3, wherein the third axis and the fourth axis are each angled relative to the first axis by a range of 5 to 30 degrees.

5. The kit of any one of claims 2-4, wherein the first stem opening is proximal to the second stem opening along a length of the stem.

6. The kit of any one of claims 2-5, wherein the first stem opening is configured to provide fixation of the proximal portion of a left femur and the second stem opening is configured to provide fixation of the proximal portion of a right femur.

7. The kit of any one of claims 1-6, wherein the bone plate (20) further comprises at least one bone screw opening (25, 26) for receiving a bone screw (60), the bone screw configured to be positioned within the femur adjacent to the stem.

8. The kit of any one of claims 1-7, wherein a proximal end of the bone plate (20) comprises a grip plate including first (23) and second prongs (24).

9. The kit of claim 8, wherein the first (23) and second prongs (24) extend proximally from the bone plate (20) and are configured to engage the outer surface of the bone.

10. The kit of any one of claims 1-9, wherein the implant (10) further comprises a distal body (11) configured to be positioned within an intramedullary canal of the femur, and the stem is configured to be positioned over a proximal end of the distal body.

11. The kit of claim 10, wherein a second locking bolt is configured to engage the stem (12, 112) to the distal body (11, 111).

12. The kit of any one of claims 1-11, further comprising a first end of a guide (30) configured to be connected to a proximal end of the stem (11) and a second end of the guide configured to be positioned adjacent to the bone plate opening (27).

13. The kit of claim 12, wherein the second end of the guide (30) is configured to position a drill (80) to ream a hole within the femur through the bone plate opening (27).

14. The kit of claim 12 or 13, wherein the second end of the guide (30) is spherical and configured to match a portion of the bone plate opening (27).

## Patentansprüche

1. Set zur Fixierung eines proximalen Teils eines Oberschenkelknochens, das Set umfassend:
einen Verriegelungsbolzen (40);
ein Implantat (10, 110), einschließlich eines Schafts (12, 112) und eines Halses (13, 113), der vom Schaft ausgeht, wobei der Hals eine längliche Ausdehnung entlang einer ersten Achse (Al) aufweist, wobei das Implantat so ausgelegt ist, dass es im proximalen Abschnitt des Oberschenkelknochens angeordnet wird,
wobei der Schaft eine Schaftöffnung (14, 15; 114, 115) zur Aufnahme des Verriegelungsbolzens einschließt, wobei die Schaftöffnung entlang einer zweiten Achse (A5) zentriert ist, die quer zur ersten Achse verläuft, und
wobei die erste Achse durch eine erste Ebene verläuft, wobei die erste Ebene durch ein erstes Ende des Schafts, ein zweites Ende des Schafts und die erste Achse definiert ist; und
eine Knochenplatte (20), die so ausgelegt ist, dass sie in eine Außenfläche des proximalen Teils des Oberschenkelknochens eingreift, wobei die Knochenplatte eine Knochenplattenöffnung (27) zur Aufnahme des Verriegelungsbolzens einschließt,
wobei der Verriegelungsbolzen so ausgelegt ist, dass er durch die Knochenplattenöffnung und in die Öffnung des Schafts hindurchgeht, um die Knochenplatte am Implantat zu befestigen,
wobei das Set **dadurch gekennzeichnet ist, dass** die zweite Achse gegenüber der ersten Achse in einem Bereich von 5 bis 30 Grad geneigt ist.

2. Set nach Anspruch 1, wobei die Schaftöffnung (14, 15; 114, 115) eine erste Schaftöffnung und eine zweite Schaftöffnung einschließt und eine dritte zur ersten Achse quer verlaufende Achse durch die erste Schaftöffnung verläuft und eine vierte zur ersten Achse quer verlaufende Achse durch die zweite Schaftöffnung verläuft.

3. Set nach Anspruch 2, wobei die dritte Achse relativ zur ersten Achse geneigt ist und die vierte Achse relativ zur ersten Achse im gleichen Winkel geneigt ist.

4. Set nach Anspruch 2 oder 3, wobei die dritte Achse und die vierte Achse jeweils in einem Winkel von 5 bis 30 Grad zur ersten Achse geneigt sind.

5. Set eines der Ansprüche 2 bis 4, wobei die erste Schaftöffnung proximal zur zweiten Schaftöffnung entlang der Länge des Schafts liegt.

6. Set eines der Ansprüche 2 bis 5, wobei die erste Schaftöffnung so ausgelegt ist, dass sie die Fixierung des proximalen Teils eines linken Oberschenkelknochens bereitstellt, und die zweite Schaftöffnung so ausgelegt ist, dass sie die Fixierung des proximalen Teils eines rechten Oberschenkelknochens bereitstellt.

7. Set eines der Ansprüche 1 bis 6, wobei die Knochenplatte (20) ferner mindestens eine Öffnung für eine Knochenschraube (25, 26) zum Aufnehmen einer Knochenschraube (60) umfasst, wobei die Knochenschraube so ausgelegt ist, dass sie innerhalb des Oberschenkelknochens neben dem Schaft positioniert wird.

8. Set eines der Ansprüche 1 bis 7, wobei ein proximaler Ende der Knochenplatte (20) eine Griffplatte umfasst, die erste (23) und zweite Zinken (24) einschließt.

9. Set nach Anspruch 8, wobei die erste (23) und zweite Zinken (24) proximal von der Knochenplatte (20) ausgehen und so ausgelegt sind, dass sie in die Außenseite des Knochens eingreifen.

10. Set eines der Ansprüche 1 bis 9, wobei das Implantat (10) ferner einen distalen Körper (11) umfasst, der so ausgelegt ist, dass er innerhalb eines intramedullären Kanals des Oberschenkelknochens positioniert wird, und der Schaft so ausgelegt ist, dass er über einem proximalen Ende des distalen Körpers positioniert wird.

11. Set nach Anspruch 10, wobei ein zweiter Verriegelungsbolzen so ausgelegt ist, dass er den Schaft (12, 112) mit dem distalen Körper (11, 111) verbindet.

12. Set eines der Ansprüche 1 bis 11, ferner umfassend ein erstes Ende einer Führung (30), die so ausgelegt ist, dass sie mit einem proximalen Ende des Schafts (11) verbunden wird, und ein zweites Ende der Führung, das so ausgelegt ist, dass es neben der Knochenplattenöffnung (27) positioniert wird.

13. Set nach Anspruch 12, wobei das zweite Ende der Führung (30) so ausgelegt ist, dass es einen Bohrer (80) positioniert, um ein Loch im Oberschenkelknochen durch die Knochenplattenöffnung (27) auszubohren.

14. Set nach Anspruch 12 oder 13, wobei das zweite Ende der Führung (30) kugelförmig ist und so ausgelegt ist, dass es einem Teil der Knochenplattenöffnung (27) entspricht.

## Revendications

1. Kit pour assurer une fixation d'une portion proximale d'un fémur, le kit comprenant :
un boulon de verrouillage (40) ;
un implant (10, 110) comprenant une tige (12, 112) et un col (13, 113) s'étendant à partir de la tige, le col ayant une dimension allongée s'étendant selon un premier axe (A1), l'implant étant configuré pour être disposé dans la portion proximale du fémur,
dans lequel la tige comporte une ouverture de tige (14, 15 ; 114, 115) pour recevoir le boulon de verrouillage, l'ouverture de tige étant centrée selon un deuxième axe (A5) transversal au premier axe, et
dans lequel le premier axe s'étend à travers un premier plan, le premier plan étant défini par une première extrémité de la tige, une deuxième extrémité de la tige et le premier axe ; et
une plaque osseuse (20) configurée pour venir en prise avec une surface externe de la portion proximale du fémur, la plaque osseuse comprenant une ouverture de plaque osseuse (27) destinée à recevoir le boulon de verrouillage,
dans lequel le boulon de verrouillage est configuré pour traverser l'ouverture de plaque osseuse et pénétrer dans l'ouverture de tige pour fixer la plaque osseuse à l'implant,
le kit étant **caractérisé en ce que** le deuxième axe forme un angle par rapport au premier axe compris entre 5 et 30 degrés.

2. Kit selon la revendication 1, dans lequel l'ouverture de tige (14, 15 ; 114, 115) comprend une première ouverture de tige et une deuxième ouverture de tige, et un troisième axe transversal au premier axe s'étend à travers la première ouverture de tige, tet un quatrième axe transversal au premier axe s'étend à travers la deuxième ouverture de tige.

3. Kit selon la revendication 2, dans lequel le troisième axe forme un angle par rapport au premier axe et le quatrième axe forme le même angle par rapport au premier axe.

4. Kit selon la revendication 2 ou 3, dans lequel le troisième axe et le quatrième axe forment chacun un angle par rapport au premier axe compris entre 5 et 30 degrés.

5. Kit selon l'une quelconque des revendications 2 à 4, dans lequel la première ouverture de tige est proximale par rapport à la deuxième ouverture de tige sur une longueur de la tige.

6. Kit selon l'une quelconque des revendications 2 à 5, dans lequel la première ouverture de tige est configurée pour assurer une fixation de la portion proximale d'un fémur gauche et la deuxième ouverture de tige est configurée pour assurer une fixation de la portion proximale d'un fémur droit.

7. Kit selon l'une quelconque des revendications 1 à 6, dans lequel la plaque osseuse (20) comprend en outre au moins une ouverture de vis osseuse (25, 26) pour recevoir une vis osseuse (60), la vis osseuse étant configurée pour être positionnée dans le fémur à proximité de la tige.

8. Kit selon l'une quelconque des revendications 1 à 7, dans lequel une extrémité proximale de la plaque osseuse (20) comprend une plaque de préhension comportant une première (23) et une deuxième (24) broches.

9. Kit selon la revendication 8, dans lequel les première (23) et deuxième (24) broches s'étendent de manière proximale à partir de la plaque osseuse (20) et sont configurées pour s'engager avec la surface externe de l'os.

10. Kit selon l'une quelconque des revendications 1 à 9, dans lequel l'implant (10) comprend en outre un corps distal (11) configuré pour être positionné dans un canal intramédullaire du fémur, et la tige est configurée pour être positionnée sur une extrémité proximale du corps distal.

11. Kit selon la revendication 10, dans lequel un deuxième boulon de verrouillage est configuré pour fixer la tige (12, 112) au corps distal (11, 111).

12. Kit selon l'une quelconque des revendications 1 à 11, comprenant en outre une première extrémité d'un guide (30) configurée pour être reliée à une extrémité proximale de la tige (11) et une seconde extrémité du guide configurée pour être positionnée à proximité de l'ouverture de plaque osseuse (27).

13. Kit selon la revendication 12, dans lequel la deuxième extrémité du guide (30) est configurée pour positionner un foret (80) pour aléser un trou dans le fémur à travers l'ouverture de plaque osseuse (27).

14. Kit selon la revendication 12 ou 13, dans lequel la deuxième extrémité du guide (30) est sphérique et configurée pour s'adapter à une portion de l'ouverture de plaque osseuse (27).
